(19) Europäisches Patentamt / European Patent Office / Office européen des brevets

(11) **EP 4 607 183 A1**

(12) **EUROPEAN PATENT APPLICATION**

(43) Date of publication:
27.08.2025 Bulletin 2025/35

(21) Application number: 25159261.4

(22) Date of filing: 21.02.2025

(51) International Patent Classification (IPC):
*G01N 23/207* (2018.01)

(52) Cooperative Patent Classification (CPC):
**G01N 23/207**; G01N 33/39

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 26.02.2024 JP 2024027017

(71) Applicant: **Rigaku Corporation**
Akishima-shi
Tokyo 196-8666 (JP)

(72) Inventors:
• **HARA, Tomomi**
Tokyo, 196-8666 (JP)
• **OGI, Aya**
Tokyo, 196-8666 (JP)
• **KOBAYASHI, Shintaro**
Tokyo, 196-8666 (JP)

(74) Representative: **Lambacher, Michael et al**
**V. Füner Ebbinghaus Finck Hano**
**Patentanwälte**
**Mariahilfplatz 3**
**81541 München (DE)**

(54) **METHOD, X-RAY DIFFRACTION SYSTEM, AND PROGRAM FOR CALCULATING MISCUT ANGLE OF SINGLE-CRYSTAL SOLID SAMPLE**

(57)  A method comprises the steps of: when a Bragg angle corresponding to the crystal lattice plane is referred to as $\theta$, making an X-ray incident on the sample at a first incident angle $\omega_1$ of $\theta-\delta$ or more and $\theta+\delta$ or less, measuring 1 or 2 peak positions of $\varphi$ by measuring a diffracted X-ray intensity while rotating the sample in-plane with the $\varphi$ as a central axis, making an X-ray incident on the sample at a second incident angle $\omega_2$ of $\theta-\delta$ or more and $\theta+\delta$ or less different from a first incident angle $\omega_1$, measuring 1 or 2 peak positions of $\varphi$ by measuring a diffracted X-ray intensity while rotating the sample in-plane with the $\varphi$ as a central axis, and calculating the $\delta$ from the $\omega_1$, the $\omega_2$ and the peak positions of the $\varphi$ obtained by the measurement.

FIG. 16

EP 4 607 183 A1

**Description**

RELATED ART

Field of the Invention

The present invention relates to a method, an X-ray diffraction system, and a program for calculating a miscut angle of a single-crystal solid sample.

Description of the Related Art

Miscut angle (offset-angle /off-angle) is often controlled for single-crystal substrates used in the semiconductor/-electronics industries. The miscut angle greatly affects the quality of the epitaxial film grown on the surface of the single crystal substrate. Therefore, control of the miscut angle is required in order to ensure and improve the device performance. Further, in addition to the miscut angle, even the azimuth angle may be controlled.

Patent Document 1 discloses a method for determining a miscut angle and an azimuth angle of a single crystal substrate by the $\varphi$ 4-point method. The $\varphi$ 4-point method is a method in which the rocking curve measurement acquired by changing the incident angle $\omega$ is executed four times for every 90° of the in-plane rotation angle $\varphi$ to calculate the miscut angle and the azimuth angle from the peak positions of the respective profiles.

Patent Document 2 discloses a method for determining a miscut angle of a single crystal substrate by $\omega$ scan while rotating at high speed in the $\varphi$ direction.

Patent Document 3 discloses a method for determining a miscut angle by measuring X-ray reflection from a plurality of crystal lattice planes during one rotation in the $\varphi$ direction.

Patent Document

Patent Document 1: JP S57-136151A
Patent Document 2: JP H03-019250A
Patent Document 3: JP2006-053141A

SUMMARY OF THE INVENTION

Patent Document 1 is a conventionally used method, but the problem is that at least four measurements are required, and the measurement takes a long time. Further, for a sample having a large variation in miscut angle and azimuth angle, the angle at which the peak is detected cannot be predicted, and it is necessary to measure a wide angle range.

In Patent Document 2, the miscut angle of the single crystal substrate can be obtained by one $\omega$ scan, but the azimuth angle cannot be obtained. Further, since the sample is rotated at a high speed, there is a problem in a method of fixing the sample and the measurement accuracy.

Patent Document 3 can determine the miscut angle of the single crystal by one $\varphi$ scan, it is necessary to prepare a mask that accepts only the X-ray reflection from a predetermined lattice plane. That is, it is a measurement method specialized for a specific single crystal and is not suitable for measurement for an arbitrary single crystal.

As a result of intensive research, the present inventors have found a method for determining a miscut angle and an azimuth angle with a small number of measurements even in a sample having a large variation in a miscut angle or an azimuth angle and have completed the present invention.

The present invention has been made in view of such circumstances, and an object thereof is to provide a method, an X-ray diffraction system, and a program for calculating a miscut angle of a single-crystal solid sample.

(1) In order to achieve the above object, the method of the present invention is a method for calculating an angle $\delta$ formed by an in-plane rotational axis $\varphi$ of a sample stage and a crystal lattice plane normal axis of a single-crystal solid sample disposed on the sample stage comprises the steps of: when a Bragg angle corresponding to the crystal lattice plane is referred to as $\theta$, making an X-ray incident on the sample at a first incident angle $\omega_1$ of $\theta$-$\delta$ or more and $\theta$+$\delta$ or less, measuring 1 or 2 peak positions of $\varphi$ by measuring a diffraction X-ray intensity while rotating the sample in-plane with the $\varphi$ as a central axis, making an X-ray incident on the sample at a second incident angle $\omega_2$ of $\theta$-$\delta$ or more and $\theta$+$\delta$ or less different from the first incident angle $\omega_1$, measuring 1 or 2 peak positions of $\varphi$ by measuring the diffracted X-ray intensity while rotating the sample in-plane with the $\varphi$ as a central axis, and calculating the $\delta$ from the $\omega_1$, the $\omega_2$ and

the peak positions of the $\varphi$ obtained by the measurement.

(2) Further, in the method of the present invention, the step of calculating the $\delta$ comprises substituting three of the combinations of $\omega_1$, $\omega_2$ and the peak positions of $\varphi$ into the following formula (1), where $\alpha$ is an azimuth angle, and solving simultaneous equations to calculate the $\delta$:

$$\cos \omega \cos(\varphi - \alpha + 180) \sin \delta + \sin \omega \cos \delta = \sin \theta \qquad \cdots \ (1)$$

(3) Further, in the method of the present invention, the step of calculating the $\delta$ comprises substituting four of the combinations of $\omega_1$, $\omega_2$ and the peak positions of $\varphi$ into the following formula (2), where $\alpha$ is an azimuth angle, and solving simultaneous equations to calculate the $\delta$:

$$\cos \omega \cos(\varphi - \alpha + 180) \sin \delta + \sin \omega \cos \delta = \sin \theta \qquad \cdots \ (2)$$

(4) Further, the method of the present invention further comprises the step of calculating the $\alpha$.

(5) Further, in the method of the present invention, the $\varphi$ coincides with the sample surface normal axis of the sample, and the $\delta$ is a miscut angle formed by a crystal lattice plane normal axis of the sample and a sample surface normal axis of the sample.

(6) Further, in the method of the present invention, the $\varphi$ coincides with the sample surface normal axis of the sample, and the method further comprises the step of tilting the sample at a predetermined angle in a predetermined direction from the state that the $\varphi$ coincides with the sample surface normal axis of the sample, the step of calculating the $\delta$ comprises using the calculated $\delta$ and $\alpha$ as a temporary value and calculating a miscut angle formed by the crystal lattice plane normal axis of the sample and the sample surface normal axis of the sample based on the $\delta$, $\alpha$, the predetermined direction and the predetermined angle.

(7) Further, the method of the present invention further comprises the step of determining a measurement and calculation method according to a user's instruction or a property of the sample.

(8) Further, the method of the present invention is a method for calculating an angle $\delta$ formed by an in-plane rotation axis $\varphi$ of a sample stage and a sample surface normal axis of a sample disposed on the sample stage, comprising the steps of: when a total reflection critical angle of the sample is referred to as $\theta c$, and a certain angle of more than 0° and the $\theta c$ or less is referred to as $\theta$, making X-rays incident on the sample at a first incident angle $\omega_1$ of $\theta$-$\delta$ or more and $\theta$+$\delta$ or less, measuring 1 or 2 peak positions of $\varphi$ by measuring the reflected X-ray intensity while rotating the sample in-plane with the $\varphi$ as a central axis, making X-rays incident on the sample at a second incident angle $\omega_2$ of $\theta$-$\delta$ or more and $\theta$+$\delta$ or less different from the first incident angle $\omega_1$, measuring 1 or 2 peak positions of the $\varphi$ by measuring the reflected X-ray intensity while rotating the sample in-plane with the $\varphi$ as the center axis, and calculating the $\delta$ from the $\omega_1$, the $\omega_2$ and the peak positions of the $\varphi$ obtained by the measurement.

(9) Further, the system of the present invention is an X-ray diffraction system comprising; an X-ray diffraction apparatus comprising an X-ray generating section for generating X-rays, a detector for detecting X-rays, a sample stage having an in-plane rotation axis $\varphi$ and a goniometer for controlling rotation of a sample, and a control apparatus comprising a control section for controlling the X-ray diffraction apparatus, a calculation section for performing calculation based on measurement data measured with the X-ray diffraction apparatus, wherein the X-ray diffraction system calculates an angle $\delta$ formed by the in-plane rotation axis $\varphi$ of the sample stage and a crystal lattice plane normal axis of a single-crystal solid sample disposed on the sample stage, when a Bragg angle corresponding to the crystal lattice plane is referred to as $\theta$, the control section makes an X-ray incident on the sample at a first incident angle $\omega_1$ of $\theta$-$\delta$ or more and $\theta$+$\delta$ or less and controls the X-ray diffraction apparatus to measure 1 or 2 peak positions of $\varphi$ by measuring the reflected X-ray intensity while rotating the sample in-plane with the $\varphi$ as a central axis, and the control section makes an X-ray incident on the sample at a second incident angle $\omega_2$ of $\theta$-$\delta$ or more and $\theta$+$\delta$ or less different from the first incident angle $\omega_1$ and controls the X-ray diffraction apparatus to measure 1 or 2 peak positions of $\varphi$ by measuring a diffracted X-ray intensity while rotating the sample in-plane with the $\varphi$ as a central axis, and the calculation section calculates the $\delta$ from the $\omega_1$, the $\omega_2$ and the peak positions of the $\varphi$ obtained by the measurement.

(10) Further, the program of the present invention is a program for calculating an angle $\delta$ formed by an in-plane rotation axis $\varphi$ of a sample stage and a crystal lattice plane normal axis of a single-crystal solid sample disposed on the sample stage by controlling an X-ray diffraction apparatus comprising an X-ray generation section for generating X-rays, a sample stage having an in-plane rotation axis $\varphi$, and a goniometer for controlling the rotation of the sample, causing a computer to execute the processes of: when a Bragg angle corresponding to the crystal lattice plane is referred to as $\theta$, making an X-ray incident on the sample at a first incident angle $\omega_1$ of $\theta$-$\delta$ or more and $\theta$+$\delta$ or less, making the X-ray diffraction apparatus measure 1 or 2 peak positions of $\varphi$ by measuring the diffracted X-rays intensity while rotating the sample in-plane with the X-ray as a central axis, making an X-ray incident on the sample at a second incident angle $\omega_2$ of $\theta$-$\delta$ or more and $\theta$+$\delta$ or less different from the first incident angle $\omega_1$, and making the X-ray diffraction apparatus measure 1 or 2 peak positions of $\varphi$ by measuring the diffracted X-ray intensity while rotating the sample in-plane with the $\varphi$ as a central axis, and calculating the $\delta$ from the $\omega_1$, the $\omega_2$ and the peak positions of the $\varphi$ obtained by the measurement.

Brief Description of the Drawings

FIG. 1 is a schematic diagram showing a cross section of a single-crystal solid sample.
FIG. 2 is a schematic diagram showing a relationship of a sample surface of a single-crystal solid sample with a miscut angle and an azimuth angle.
FIG. 3 is a schematic diagram showing a precession of a crystal lattice plane normal.
FIG. 4 is a graph showing precession of the crystal lattice plane normal.
FIG. 5 is a schematic graph showing a relationship between peaks of diffracted X-rays with respect to an incident angle $\omega_0$ and a graph corresponding to precession of a crystal lattice plane normal.
FIG. 6 is a graph showing the result of a $\varphi$ scan for a plurality of $\omega$ values as a vertical axis offset.
FIG. 7 is a schematic diagram showing a single-crystal solid sample with an offset disposed on a sample stage and respective axes.
FIG. 8 is a schematic graph showing a relationship between a magnitude relationship of $\varphi_1$ to $\varphi_4$ and a graph corresponding to precession of a crystal lattice plane normal.
FIG. 9 is a schematic graph showing a relationship between a magnitude relationship of $\varphi_1$ to $\varphi_4$ and a graph corresponding to precession of a crystal lattice plane normal.
FIG. 10 is a schematic graph showing a relationship between a magnitude relationship of $\varphi_1$ to $\varphi_4$ and a graph corresponding to precession of a crystal lattice plane normal.
FIG. 11 is a schematic graph showing a relationship between a magnitude relationship of $\varphi_1$ to $\varphi_4$ and a graph corresponding to precession of a crystal lattice plane normal.
FIG. 12 is a conceptual diagram showing one example of the configuration of the system for an X-ray diffraction measurement.
FIG. 13 is a block diagram showing one example of the configuration of the control apparatus.
FIGS. 14A and 14B are schematic diagrams showing UI examples of entry dialogues when the software is executed.
FIG. 15 is a schematic diagram showing an example of a measurement result output screen of the software.
FIG. 16 is a flowchart showing an example of the operation of the X-ray diffraction system.
FIG. 17 is a flowchart showing a modified example of the operation of the X-ray diffraction system.
FIG. 18 is a flowchart showing a modified example of the operation of the X-ray diffraction system.
FIG. 19 is a flowchart showing a modified example of the operation of the X-ray diffraction system.
FIG. 20 is a table showing the values of the pseudo offset and the calculation results of the miscut angle and the azimuth angle calculated by the method of the present invention.
FIG. 21 is a table showing the values of the pseudo offset and the calculation results of the miscut angle and the azimuth angle calculated by the $\varphi$ 4-point method.

DETAILED DESCRIPTION OF THE INVENTION

Next, embodiments of the present invention are described with reference to the drawings. To facilitate understanding of the description, the same reference numerals are assigned to the same components in the respective drawings, and duplicate descriptions are omitted.

[Principle]

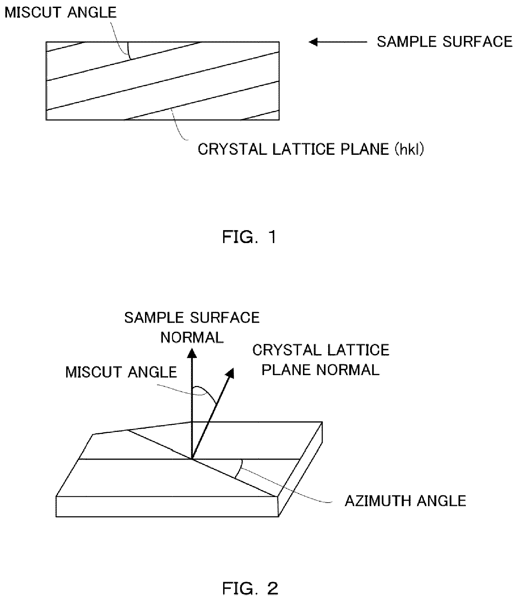

FIG. 1 is a schematic diagram showing a cross section of a sample having crystal-oriented (hereinafter, referred to as a single-crystal solid sample). FIG. 1 shows a cross section perpendicular to the surface and the crystal lattice plane of a single-crystal solid sample. As shown in FIG. 1, a miscut angle of the single-crystal solid sample refers to an angle formed between the sample surface and the crystal lattice plane. FIG. 2 is a schematic diagram showing a relationship of a sample surface of a single-crystal solid sample with a miscut angle and an azimuth angle. As shown in FIG. 2, the miscut angle of the single-crystal solid sample appears as an angle formed by the sample surface normal and the crystal lattice plane normal. Further, an azimuth angle of the single-crystal solid sample is an angle formed between the projection of the crystal lattice plane normal to the sample surface and one direction set on the sample surface.

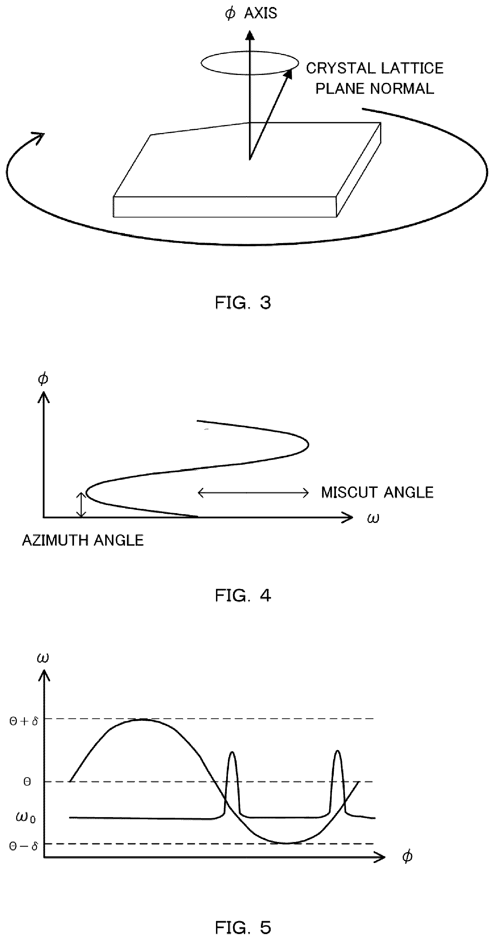

When the crystal lattice plane normal of the single-crystal solid sample disposed on the sample stage of the X-ray diffraction apparatus do not coincide with the in-plane rotation axis $\varphi$ of the sample stage, the crystal lattice plane normal performs precession when the sample is rotated in-plane. FIG. 3 is a schematic diagram showing a precession of a crystal lattice plane normal. FIG. 4 is a graph showing precession of the crystal lattice plane normal. The precession of the crystal lattice plane normal appears as a sin like curve. If a graph of precession of such a crystal lattice plane normal can be obtained, the miscut angle and the azimuth angle can be obtained. In practice, since the graph of the precession of the crystal lattice surface normal is unknown, it is considered that the graph of the precession of the crystal lattice surface normal is obtained using the measurement data of the diffraction intensity.

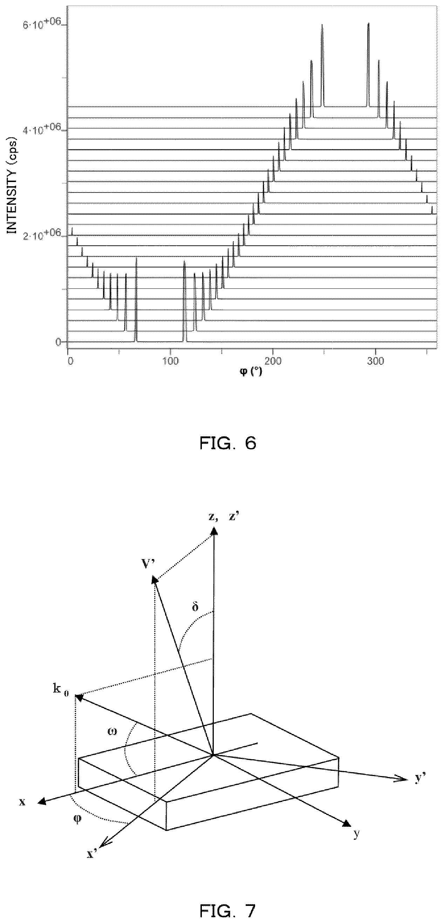

Here, when a $\varphi$ scan is performed with fixing the incident angle $\omega$ of the X-ray to a certain angle $\omega_0$, the diffraction lines are detected when the angle formed by the incident X-ray and the crystal lattice plane normal is $\theta$, and two peaks are obtained. The $\varphi$ scan is a method of measuring the X-ray diffraction intensity while rotating the sample in-plane with $\varphi$ as an axis in a state in which the incident angle $\omega$ of the X-ray is fixed. Further, $\theta$ is a Bragg angle of reflection of the crystal lattice plane. That is, when the $\varphi$ scan is performed, two peaks are obtained at positions corresponding to precession of the crystal lattice plane normal. FIG. 5 is a schematic graph showing a relationship between peaks of diffracted X-rays with respect to an incident angle $\omega_0$ and a graph corresponding to precession of a crystal lattice plane normal. $\delta$ is the miscut angle. When the $\varphi$ scan is repeated with changing $\omega$, the peak position changes along the sin like curve and moves continuously. FIG. 6 is a graph showing the result of a $\varphi$ scan for a plurality of $\omega$ values as a vertical axis offset for a Si substrate. Si substrate used is a Si (100) substrate with a miscut angle of about 3°, and Si 004 reflection was used for the measurement. The value of $\omega$ was selected in 0.25° steps, ranging from $\theta$-2.75° to $\theta$+2.75°. As described above, by performing the $\varphi$ scan in a wide $\omega$ range, curves corresponding to precessions of the crystal lattice plane normal as shown in FIG. 5 are finally obtained.

In practice, the miscut angle and the azimuth angle can be determined by calculation using two $\varphi$ scans with different incident angles $\omega$ and the values of their peak positions. That is, the method of the present invention is a method of performing a $\varphi$ scan with two $\omega$ values based on such a principle, estimating a shape of a curve from the obtained peak positions, and calculating the miscut angle and the azimuth angle. The detailed method according to the present invention is described in the embodiment.

[Embodiment]

The method according to the present invention is explained in detail, as described below. Hereinafter, an example of a method of calculating the miscut angle and the azimuth angle using data of peak positions when two $\varphi$ scans with different incident angles $\omega$ are performed with an X-ray diffraction apparatus is described. However, the method described below is merely an example, and the used mathematical expressions, the order of calculations or the method may be different from that in the example. In addition, the method of the present invention includes a method of calculating a miscut angle or an azimuth angle using data of a peak position when two $\varphi$ scans with different incident angles $\omega$ are performed in an X-ray diffraction apparatus. The present methods can be used to calculate the miscut angle or the azimuth angle of a single crystal substrate or an epitaxial film made of such as Si, sapphire, GaN, GaAs and diamond.

In the following description, it is assumed that the in-plane rotation axis $\varphi$ of the sample stage coincides with the sample surface normal axis of the sample. FIG. 7 is a schematic diagram showing a single-crystal solid sample with an offset disposed on a sample stage and respective axes. A normal vector of a crystal lattice plane having a crystal lattice plane offset (deviation from the sample surface) is referred to as a vector V. When the vector V is inclined $\delta$ in the x-direction from the z-direction, the vector V is expressed by the following formula (3). Since the in-plane rotation axis $\varphi$ of the sample stage coincides with the sample surface normal axis of the sample, the tilt $\delta$ is a miscut angle formed by the crystal lattice plane normal axis of the sample and the sample surface normal axis of the sample.

$$\vec{V} = \begin{pmatrix} \sin\delta \\ 0 \\ \cos\delta \end{pmatrix} \qquad \cdots \ (3)$$

When it is assumed that (x', y', z') is obtained by rotating (x,y,z) around the $\varphi$-axis (z-axis in FIG. 7), the relationship between (x,y,z) and (x', y', z') is expressed by multiplication with a three-dimensional rotational matrix as in formula (4) below.

$$\begin{pmatrix} x' \\ y' \\ z' \end{pmatrix} = \begin{pmatrix} \cos\varphi & \sin\varphi & 0 \\ -\sin\varphi & \cos\varphi & 0 \\ 0 & 0 & 1 \end{pmatrix} \begin{pmatrix} x \\ y \\ z \end{pmatrix} \qquad \cdots \ (4)$$

Therefore, when a vector obtained by rotating the vector V around the $\varphi$-axis is referred to as a vector V', the vector V' is expressed by the following formula (5).

$$\vec{V'} = \begin{pmatrix} \cos\varphi & \sin\varphi & 0 \\ -\sin\varphi & \cos\varphi & 0 \\ 0 & 0 & 1 \end{pmatrix} \begin{pmatrix} \sin\delta \\ 0 \\ \cos\delta \end{pmatrix} = \begin{pmatrix} \cos\varphi\sin\delta \\ -\sin\varphi\sin\delta \\ \cos\delta \end{pmatrix} \qquad \cdots (5)$$

On the other hand, when it is assumed that the incident angle of the incident X-ray $k_0$ on xz plane is referred to as $\omega$, the vector $k_0$ is expressed by the following formula (6).

$$\vec{k_0} = \begin{pmatrix} \cos\omega \\ 0 \\ \sin\omega \end{pmatrix} \qquad \cdots (6)$$

The Bragg angle of the reflection by the crystal lattice plane of interest in the single-crystal solid sample is defined as $\theta$. When the normal vector V' of the crystal lattice plane rotated by a certain angle $\varphi$ satisfies the diffraction condition for the incident X-ray $k_0$, the angle formed by the vector V' and the vector $k_0$ is expressed as $(\pi/2-\theta)$. When it is assumed that the angle formed by the vector a and the vector b is referred to as $\beta$, the inner product of the vector a and the vector b is expressed by the following formula (7).

$$\vec{a} \cdot \vec{b} = |a||b|\cos\beta \qquad \cdots (7)$$

Therefore, the inner product of the vector $k_0$ and the vector V' is expressed by formula (8) below. Provided that both were defined as length 1.

$$\vec{k_0} \cdot \vec{V'} = |k_0||V|\cos\left(\frac{\pi}{2} - \theta\right) = \sin\theta \qquad \cdots (8)$$

On the other hand, from formula (3) and formula (6), the inner product of the vector $k_0$ and the vector V' is also expressed as formula (9) below.

$$\vec{k_0} \cdot \vec{V'} = \begin{pmatrix} \cos\omega \\ 0 \\ \sin\omega \end{pmatrix} \cdot \begin{pmatrix} \cos\varphi\sin\delta \\ -\sin\varphi\sin\delta \\ \cos\delta \end{pmatrix} = \cos\omega\cos\varphi\sin\delta + \sin\omega\cos\delta \qquad \cdots (9)$$

From formula (8) and formula (9), the following formula (10) can be derived.

$$\cos\omega\cos\varphi\sin\delta + \sin\omega\cos\delta = \sin\theta \qquad \cdots (10)$$

When formula (10) is modified so that $\varphi=\alpha$ and $\omega$ is the maximum value at $\varphi=\alpha$ ($\alpha$=azimuth angle), the following formula (11) is obtained.

$$\cos\omega\cos(\varphi - \alpha + 180)\sin\delta + \sin\omega\cos\delta = \sin\theta \qquad \cdots (11)$$

Here, $\varphi$ scan is performed at the incident angle $\omega_1$, and the obtained peak positions of $\varphi$ are referred to as $\varphi_1$, $\varphi_2$ ($\varphi_1 < \varphi_2$). In addition, the peak positions of $\varphi$ obtained at the incident angle $\omega_2$ ($\omega_1 > \omega_2$) are referred to as $\varphi_3$, $\varphi_4$ ($\varphi_3 < \varphi_4$). Here, $\omega_1$ and $\omega_2$ are set to $\theta$-$\delta$ or more and $\theta$+$\delta$ or less. Then, from formula (11), the following formulas (12) to (15) are obtained. Note that the peak positions of $\varphi$ are values of $\varphi$ indicating the peaks of the X-ray intensity obtained from the profile of the measurement data of the $\varphi$ scan.

$$\cos\omega_1\cos(\varphi_1 - \alpha + 180)\sin\delta + \sin\omega_1\cos\delta = \sin\theta \qquad \cdots (12)$$

$$\cos\omega_1\cos(\varphi_2 - \alpha + 180)\sin\delta + \sin\omega_1\cos\delta = \sin\theta \qquad \cdots (13)$$

$$\cos \omega_2 \cos(\varphi_3 - \alpha + 180) \sin \delta + \sin \omega_2 \cos \delta = \sin \theta \qquad \cdots (14)$$

$$\cos \omega_2 \cos(\varphi_4 - \alpha + 180) \sin \delta + \sin \omega_2 \cos \delta = \sin \theta \qquad \cdots (15)$$

Among the above formulas (12) to (15), the following formula (16) can be generated by using, for example, the formulas (12) and (14). When both sides of formula (16) are divided by $\cos\delta$, formula (17) below is obtained. This is transformed to give the following formula (18).

$$\cos \omega_1 \cos(\varphi_1 - \alpha + 180) \sin \delta + \sin \omega_1 \cos \delta$$

$$= \cos \omega_2 \cos(\varphi_3 - \alpha + 180) \sin \delta + \sin \omega_2 \cos \delta \qquad \cdots (16)$$

$$\cos \omega_1 \cos(\varphi_1 - \alpha + 180) \tan \delta + \sin \omega_1$$

$$= \cos \omega_2 \cos(\varphi_3 - \alpha + 180) \tan \delta + \sin \omega_2 \qquad \cdots (17)$$

$$\delta = \tan^{-1}\left\{ \frac{\sin \omega_1 - \sin \omega_2}{-\cos \omega_1 \cos(\varphi_1 - \alpha + 180) + \cos \omega_2 \cos(\varphi_3 - \alpha + 180)} \right\} \qquad \cdots (18)$$

Similar mathematical expressions can be generated by a combination of either one of formulas (12) and (13) and either one of formulas (14) and (15).

In addition, since the profile of the measurement data of the $\varphi$ scan is symmetrical about the peak/valley, the azimuth angle $\alpha$ can be calculated using $\varphi_1$, $\varphi_2$, $\varphi_3$ and $\varphi_4$. $\omega_1$, $\omega_2$, $\varphi_1$, $\varphi_2$, $\varphi_3$ and $\varphi_4$ are defined as above. For example, $\alpha$ can be calculated by case classification of the magnitude relation of $\varphi_1$ to $\varphi_4$ as follows. Note that $\alpha$ may be a negative value.

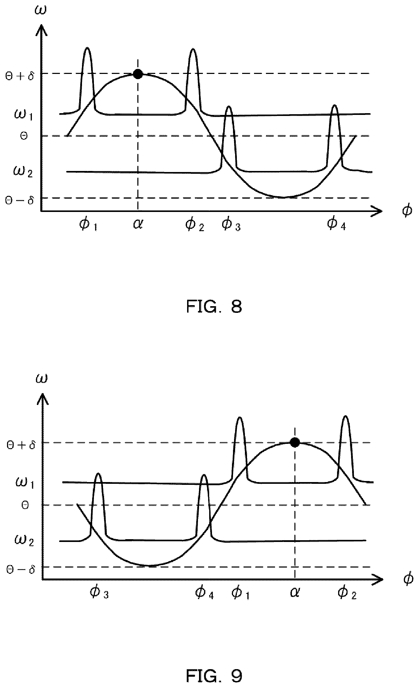

FIG. 8 to FIG. 11 are schematic graphs showing relationships between the magnitude relationship of $\varphi_1$ to $\varphi_4$ and the graph corresponding to precession of a crystal lattice plane normal. In FIG. 8 to FIG. 11, the incident angle $\omega_1$ is in a range larger than $\theta$, and the incident angle $\omega_2$ is in a range smaller than $\theta$, but the positions of $\omega_1$ and $\omega_2$ are not limited thereto.

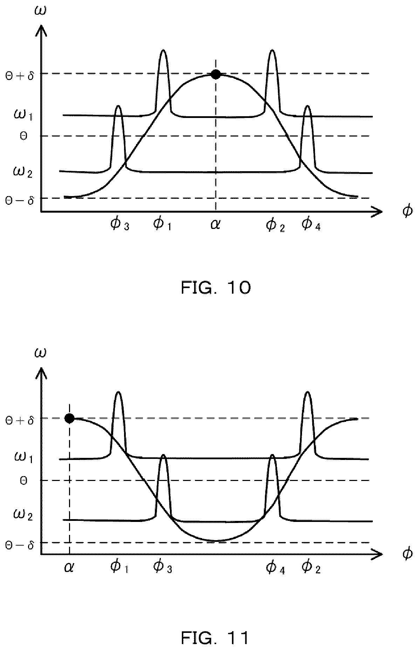

As shown in FIG. 8, for $\varphi_1 < \varphi_2 < \varphi_3 < \varphi_4$, $\alpha$ can be calculated by, for example, $\alpha=\{(\varphi_1+\varphi_2+\varphi_3+\varphi_4)/4-90\}°$. As shown in FIG. 9, for $\varphi_3 < \varphi_4 < \varphi_1 < \varphi_2$, $\alpha$ can be calculated by, for example, $\alpha=\{(\varphi_1+\varphi_2+\varphi_3+\varphi_4)/4+90\}°$. As shown in FIG. 10, for $\varphi_3 < \varphi_1 < \varphi_2 < \varphi_4$, $\alpha$ can be calculated by, for example, $\alpha=\{(\varphi_1+\varphi_2+\varphi_3+\varphi_4)/4\}°$. As shown in FIG. 11, for $\varphi_1 < \varphi_3 < \varphi_4 < \varphi_2$, $\alpha$ can be calculated by, for example, $\alpha=\{(\varphi_1+\varphi_2+\varphi_3+\varphi_4)/4-180\}°$.

Although the above described calculation of $\alpha$ uses all $\varphi_1$ to $\varphi_4$ in order to reduce the effects of measurement errors and the like, since it is $\omega_1 > \omega_2$, for example, when $\varphi_1 < \varphi_2 < \varphi_3 < \varphi_4$, it is also possible to calculate $\alpha$ as $\alpha=\{(\varphi_1+\varphi_2)/2\}°$. That is, although it is unnecessary to use all of $\varphi_1$ to $\varphi_4$ in order to calculate $\alpha$, it is possible to reduce the effects of measurement errors and the like by using all of them. The same applies to $\delta$.

$\delta$ can be calculated by substituting several values of $\varphi_1$ to $\varphi_4$ and values of $\alpha$ calculated as described above into formula (18) or a similar mathematical expression. In addition, by generating a mathematical expression in which $\theta$ is eliminated as in formula (18) or a similar mathematical expression, the calculation amount can be reduced when it is not necessary to calculate $\theta$ of the actual measurement value. In the above described method, $\delta$ is calculated after $\alpha$ is calculated, but $\delta$ may be calculated without calculating $\alpha$. Alternatively, $\alpha$ may be calculated after $\delta$ is calculated.

As can be seen from formula (11), since the number of unknowns when calculating $\delta$ or $\alpha$ is three of $\delta$, $\alpha$, and $\theta$. Therefore, $\delta$ and $\alpha$ can be calculated by using three of the combinations of $\omega_1$, $\omega_2$ and the peak positions of $\varphi$. In addition, when four of the combinations of $\omega_1$, $\omega_2$ and the peak positions of $\varphi$ are used, an over-determination system occurs. However, since an error is included in the measured value, the effect of the measurement error or the like can be reduced by using four of the combinations, and the calculated $\delta$ or $\alpha$ can be brought close to the true value. When four of the combinations are used, any method for calculating the final value may be used. For example, $\delta$ is respectively calculated with both formula (18) generated using formulas (12) and (14) and a mathematical expression similar to formula (18) generated using formulas (13) and (15), and the average value of the two values may be the final value of $\delta$. Further, for example, a method using four mathematical expressions similar to formula (18) may be used.

The above example shows a solving method for the simultaneous equations of formula (11) using the case classification with values of $\varphi_1$, $\varphi_2$, $\varphi_3$ and $\varphi_4$. However, the solving method of the simultaneous equations may be one to be generally used, and the method of the present invention is not limited to the above described method. For example, there is a method of solving simultaneous equations by numerical calculation such as Newton's method for three unknowns $\delta$, $\alpha$, and $\theta$. When numerical calculation is used, the unknowns can be obtained without the case classification with values of $\varphi_1$, $\varphi_2$, $\varphi_3$ and $\varphi_4$. Furthermore, it is also possible to combine the case classification and the numerical calculation. For example, when the value of $\alpha$ is determined in cases and then other variables are calculated numerically, the number of unknowns is reduced, so that the amount of calculation can be reduced.

Further, in FIG. 5, when the incident angle $\omega$ approaches $\theta+\delta$ or $\theta-\delta$, two peaks of $\varphi$ with respect to $\omega$ approach each other to detect one peak at last. Even in such a case, $\delta$ and $\alpha$ can be determined. For example, when the peak position of $\varphi$ with respect to $\omega_1$ becomes one, $\delta$ and $\alpha$ can be calculated because there are three combinations of the peak positions of $\omega_1$, $\omega_2$ and $\varphi$ for $\varphi_1=\varphi_2$. The same applies to $\varphi_3$ and $\varphi_4$. Furthermore, even when the peak position of $\varphi$ with respect to $\omega_1$ is one and the peak position of $\varphi$ with respect to $\omega_2$ is one, $\delta$ and $\alpha$ can be calculated because a new condition is generated in which the angle between $\varphi_1=\varphi_2$ and $\varphi_3=\varphi_4$ is 180°.

Note that when the incident angle $\omega$ approaches $\theta$, the peak intensity of $\varphi$ becomes weak, and the measurement error of the peak position of $\varphi$ may become large. On the other hand, when the incident angle $\omega$ approaches $\theta+\delta$ or $\theta-\delta$, either of $\varphi_1$ to $\varphi_4$ approaches each other, and the calculation error may increase.

In FIG. 5, when the incident angle $\omega$ exceeds $\theta+\delta$ or falls below $\theta-\delta$, the peak of $\varphi$ is not detected when the $\varphi$ scan is performed. Here, as the Bragg angle $\theta$ for the reflection by the crystal lattice plane of interest in the single-crystal solid sample, a theoretical value can be found by using the information of the crystal of the single-crystal solid sample. Since there is little difference between the theoretical value of $\theta$ and the measured value of $\theta$, there is no problem with $\theta$. On the other hand, the angle $\delta$ formed by the in-plane rotation axis $\varphi$ of the sample stage and the crystal lattice plane normal axis of the single-crystal solid sample disposed on the sample stage is a value that is to be determined in the future and is an unknown value. When $\delta$ is very small and the in-plane rotation axis $\varphi$ of the sample stage is aligned with the sample surface normal axis of the sample, there is a possibility that the incident angle $\omega$ exceeds $\theta+\delta$ or falls below $\theta-\delta$.

In such a case, first, the sample is inclined at a predetermined angle in a predetermined direction from a state in which the in-plane rotation axis $\varphi$ of the sample stage coincides with the sample surface normal axis of the sample. Next, as described above, the $\varphi$ scan is performed at each of the incident angles $\omega_1$ and $\omega_2$. $\omega_1$ and $\omega_2$ are set within $\theta\pm$(a predetermined angle). Next, $\delta$ and $\alpha$ are determined. $\delta$ and $\alpha$ obtained at this time are temporary values. Then, the actual miscut angle or azimuth angle is calculated by using $\delta$, $\alpha$, the predetermined direction and the predetermined angle. Since the predetermined direction and the predetermined angle in which the sample is tilted are known, the actual miscut angle or azimuth angle can be calculated by using $\delta$, $\alpha$, the predetermined direction and the predetermined angle.

Specifically, for example, the actual miscut angle or azimuth angle of the sample can be calculated by the following method. The actual miscut angle of the sample is referred to as $\delta$ and the azimuth angle is referred to as $\alpha$. Next, the sample is tilted by an $\gamma_{tilt}$ toward the direction at 0° of $\varphi$-axis, and the apparent miscut angle is increased. Next, a $\varphi$ scan is performed with the apparent miscut angle increased, and the obtained miscut angle is referred to as $\delta_M$ and the azimuth angle is referred to as $\alpha_M$. At this time, the actual miscut angle $\delta$ and the azimuth angle $\alpha$ of the sample can be calculated by the following formula (19).

$$\begin{pmatrix} \cos\alpha\sin\delta \\ -\sin\alpha\sin\delta \\ \cos\delta \end{pmatrix} = \begin{pmatrix} -\cos\gamma_{tilt} & 0 & -\sin\gamma_{tilt} \\ 0 & 1 & 0 \\ -\sin\gamma_{tilt} & 0 & -\cos\gamma_{tilt} \end{pmatrix} \begin{pmatrix} \cos\alpha_M\sin\delta_M \\ -\sin\alpha_M\sin\delta_M \\ \cos\alpha_M \end{pmatrix} \quad \cdots \quad (19)$$

In this way, the miscut angle and the azimuth angle can be calculated using the data of the peak positions of the diffracted X-ray intensity from two $\varphi$ scans with different incident angles $\omega$ performed with the X-ray diffraction apparatus.

As described above, the method of the present invention can calculate $\delta$ or $\alpha$ by measuring the diffracted X-ray intensity by two $\varphi$ scans. The method of the present invention can be applied to the surface alignment of a sample placed on the sample stage by applying it to the reflected X-ray intensity.

It is assumed that the in-plane rotation axis $\varphi$ of the sample stage does not coincide with the sample surface normal axis of the sample disposed on the sample stage, the angle formed between them is referred to as $\delta$ and the tilt direction of the sample surface normal axis is referred to as $\alpha$. Further, the total reflection critical angle of the sample is referred to as $\theta c$, and a specific angle greater than 0° and $\theta c$ or less is referred to as $\theta$. With the X-ray diffraction apparatus, two $\varphi$-scans at different incident angle $\omega_1$ and $\omega_2$ with $\theta-\delta$ or more and $\theta+\delta$ or less are performed, and the reflected X-ray intensity is measured to specify 1 or 2 peak positions of $\varphi$, respectively. Then, $\delta$ and $\alpha$ are calculated using the peak position of $\varphi$ with respect to $\omega_1$ and the peak position of $\varphi$ with respect to $\omega_2$. Since the tilt of the sample can be adjusted using the calculated $\delta$ and $\alpha$, the sample surface normal axis can coincide with the in-plane rotation axis $\varphi$ of the sample stage.

X-rays incident to the sample at an angle of the total reflection critical angle or less are reflected symmetrically on the sample surface. In a state where the in-plane rotation axis $\varphi$ of the sample stage does not coincide with the sample surface normal axis of the sample disposed on the sample stage, when the $\varphi$ scan is performed, since the angle formed by the sample surface normal axis and the incident X-ray changes, the direction in which the reflected X-ray is obtained changes. 1 or 2 peak positions of φ can be measured by disposing a detector at a specified 2θ angle and detecting reflected X-rays in advance.

The procedure for adjusting the surface of the sample is the same as the procedure for determining the miscut angle, and the solution of the simultaneous equations and simultaneous equations to be solved is also the same, and therefore, the description thereof is omitted. In this way, the data of the peak position of the reflected X-ray intensity is used, when two φ scans with different incident angles ω can be used to adjust the surface of the sample placed on the sample stage with the X-ray diffraction apparatus.

The measurement method by the φ 4-point method is also a method conventionally used in the surface adjustment of a sample. However, in the φ 4-point method, at least four measurements are required, and the measurement takes time. Using the method of the present invention, it is possible to perform the surface adjustment of the sample with a small number of measurements, it is expected to reduce the measurement time.

[X-ray Diffraction System]

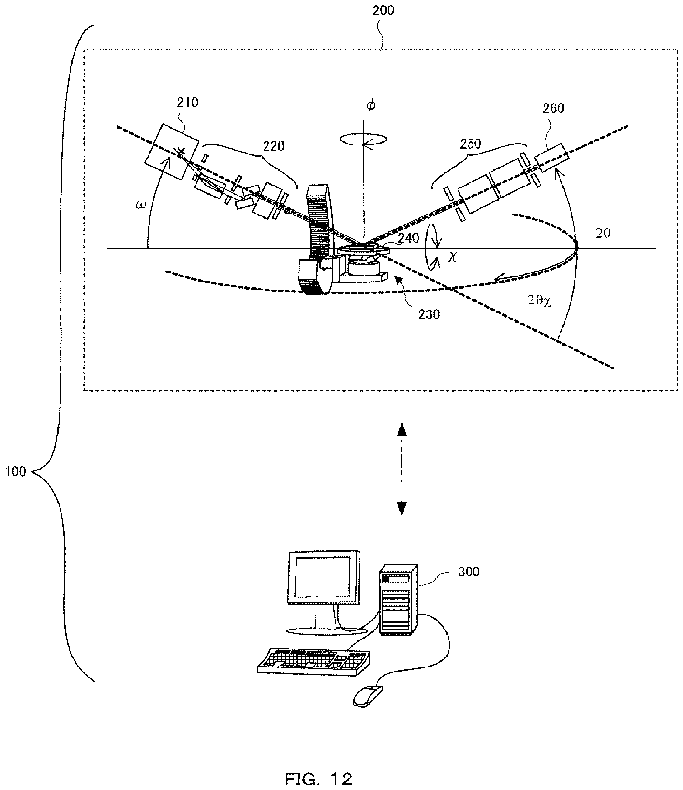

FIG. 12 is a conceptual diagram showing an example of the configuration of the X-ray diffraction system 100 of the present invention. The X-ray diffraction system 100 comprises an X-ray diffraction apparatus 200 and a control apparatus 300. The X-ray diffraction apparatus 200 makes X-rays incident on a sample and constitutes an optical system for detecting diffracted X-rays generated from the sample, and the optical system comprises a goniometer. In addition, the configuration shown in FIG. 12 is one example, and thus a variety of other configurations may be adopted.

The control apparatus 300 is connected to the X-ray diffraction apparatus 200 to control the X-ray diffraction apparatus 200 and process, store, and calculate a miscut angle or azimuth angle of the acquired data. The control apparatus 300 is an apparatus comprising CPU and a memory and may be a PC terminal or a server on a cloud. Not only the whole apparatus but also part of the apparatus or some functions of the apparatus may be provided on the cloud. The input device 510 is, for example, a keyboard or a mouse, and performs input to the control apparatus 300. The display device 520 is, for example, a display, and displays measurement data, a miscut angle, a calculation result of an azimuth angle and the like.

By using such a system, the miscut angle and azimuth angle can be calculated using the data of the peak positions of the diffracted X-ray intensity when the X-ray diffraction apparatus performs two φ scans with different incident angles ω.

[X-ray Diffraction Apparatus]

The X-ray diffraction apparatus 200 comprises an X-ray generating section 210 that generates X-rays from an X-ray focal point or X-ray source, a goniometer 230 that controls rotation of the sample, a sample stage 240 that has an in-plane rotation axis φ and installs the sample and a detector 260 that detects X-rays. The X-ray diffraction apparatus 200 may comprise an incident-side optical unit 220 and an emitting side optical unit 250. Since the X-ray generation section 210, the incident-side optical unit 220, the goniometer 230, the sample stage 240, the emitting side optical unit 250 and the detector 260 constituting the X-ray diffraction apparatus 200 need only be general, the description thereof is omitted.

[Control Apparatus]

The control apparatus 300 is constituted from a computer formed by connecting CPU (Central Processing Unit/Central Processor), ROM (Read Only Memory), RAM (Random Access Memory) and a memory to a bus. The control apparatus 300 is connected to the X-ray diffraction apparatus 200 to receive information.

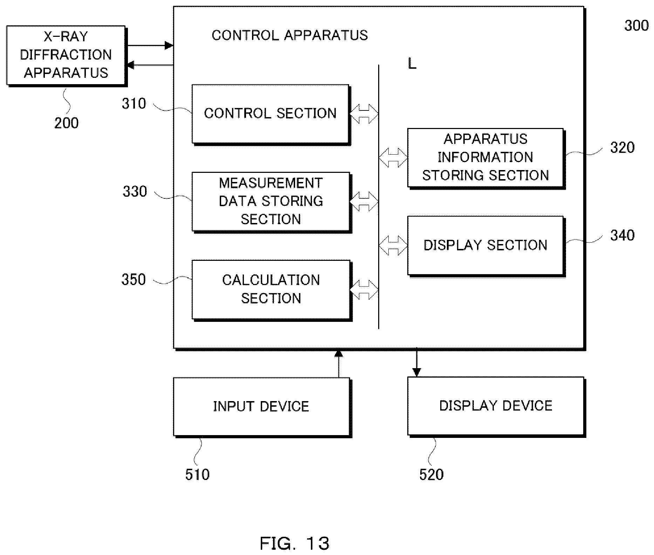

FIG. 13 is a block diagram showing one example of the configuration of the control apparatus 300. The control apparatus 300 comprises a control section 310 and a calculation section 350. The control apparatus 300 may comprise an apparatus information storing section 320, a measurement data storing section 330 or a display section 340. Each section can transmit and receive information via the control bus L. The input device 510 and the display device 520 are connected to CPU via an appropriate interface.

The control section 310 controls the operations of the X-ray diffraction apparatus 200. The control section 310 controls the X-ray diffraction apparatus 200 to measure 1 or 2 peak positions of φ by causing the X-ray to enter the sample at a first incident angle $\omega_1$ of θ-δ or more and θ+δ or less, rotating the sample in-plane with φ as a central axis and measuring the diffracted X-ray intensity. In addition, the control section 310 controls the X-ray diffraction apparatus 200 to measure 1 or 2 peak positions of φ by causing X-rays to enter the sample at a second incident angle $\omega_2$ of θ-δ or more and θ+δ or less that differs from the first incident angle $\omega_1$, rotating the sample in-plane with φ as a central axis and measuring the diffracted X-ray intensity.

It is preferable that the control section 310 controls the X-ray diffraction apparatus 200 to tilt the sample stage 240 at a predetermined angle in a predetermined direction to make a measurement. Thus, even when the miscut angle of the sample is small, the miscut angle or the azimuth angle can be calculated.

The control section 310 preferably determines whether to apply the measurement and calculation method of the present invention according to the user's instruction or the property of the sample and then determines the measurement and calculation method. The property of the sample is, for example, whether or not the miscut angle is large enough to be calculated by the method of the present invention, whether or not the variation in the miscut angle and the azimuth angle is large among a plurality of samples of the same type, and the like. The determination of the measurement and calculation method may be automatically determined by the control section acquiring the property of the sample.

In such cases that the variation of the miscut angle and the azimuth angle among a plurality of samples of the same type is large, and that the sample has a miscut angle larger than the predicted miscut angle, it is possible to greatly shorten the measurement time by the method of the present invention compared with the conventional method. In the conventional $\varphi$ 4-point method, when the variation in the miscut angle is large, the scan range of the incident angle $\omega$ needs to be large, and the scan time also is long. In the present invention, the case where the variation of the miscut angle is large means a state in which the measurement time in the conventional $\varphi$ 4-point method exceeds the measurement time of the present invention. Therefore, by selecting the measurement and calculation method according to the properties of the sample as described above, it is possible to appropriately reduce the total time for calculating the miscut angle for each of the plurality of samples.

The apparatus information storing section 320 stores apparatus information acquired from the X-ray diffraction apparatus 200. The apparatus information includes information about the X-ray diffraction apparatus 200 such as name of the apparatus, the kind of a radiation source, a wavelength, a background and so forth. In addition, information necessary for calculating the miscut angle or the azimuth angle from the measurement data of the X-ray diffraction, such as the type of the constituent elements, composition and crystal structure of the sample may be included. The apparatus information storing section 320 may store a program for controlling the X-ray diffraction apparatus 200. Further, the apparatus information storing section 320 may store a program, a mathematical expression, a numerical value, a condition and the like necessary for calculating the miscut angle or the azimuth angle from the measurement data of the X-ray diffraction. These may be stored in the measurement data storing section 330 of the control apparatus 300 or a storage section different therefrom.

The measurement data storing section 330 stores the measurement data acquired from the X-ray diffraction apparatus 200. The measurement data may include information necessary for calculating the miscut angle or the azimuth angle from the measurement data of the X-ray diffraction, such as the type of the ray source, the wavelength, the background, the type of constituent elements and the composition of the sample, and the like.

The display section 340 causes the display device 520 to display the measurement data and the calculated miscut angle or azimuth angle. Thus, the measurement data, miscut angle and azimuthal angle can be confirmed by a user. Further, instruction and designation to the control apparatus 300 and so forth can be made based on the measurement data by the user.

The calculation section 350 calculates the miscut angle or azimuth angle based on the measurement data. The calculation section 350 calculates $\delta$ or $\alpha$ from the measurement $\omega_1$, $\omega_2$ and the peak positions of $\varphi$. The calculation section 350 may be implemented as a function of an apparatus different from the control apparatus 300.

The calculation section 350 can calculate $\delta$ or $\alpha$ by substituting three of the combinations of $\omega_1$, $\omega_2$ and the peak positions of $\varphi$ into the above formula (11) and solving the simultaneous equations. The calculation of $\delta$ or $\alpha$ is facilitated by preparing a mathematical expression such as formula (11) in advance and storing it in the calculation section 350. Moreover, since it is not an overdetermined system, the calculation result is determined to be one.

The calculation section 350 can calculate $\delta$ or $\alpha$ by substituting four of the combinations of $\omega_1$, $\omega_2$ and the peak positions of $\varphi$ into the above formula (11) and solving the simultaneous equations. Thus, the effect of measurement error or the like can be reduced, and the calculated $\delta$ or $\alpha$ can be brought close to a true value.

When the measurement data is data obtained by measuring with tilting the sample stage 240 at a predetermined angle in a predetermined direction, the calculation section 350 preferably calculates $\delta$ and $\alpha$ as temporary values and calculates a miscut angle or an azimuth angle based on $\delta$, $\alpha$, a predetermined direction and a predetermined angle. Thus, even when the miscut angle of the sample is small, the miscut angle or the azimuth angle can be calculated.

In a case where the measurement and calculation method of the present invention are not applied, the calculation section 350 preferably calculates a miscut angle or azimuth angle based on another measurement and calculation method. As another measurement and calculation method, for example, the $\varphi$ 4-point method can be applied.

[User Interface]

When a user instructs the measurement conditions and the like to the X-ray diffraction system 100, it is preferable to use a user interface (UI) function that allows various settings to be made by, for example, a mouse-operation or a keyboard-operation. In addition, the X-ray diffraction system 100 is preferably configured such that the functions of the X-ray diffraction apparatus 200 and the control apparatus 300 associate with each other. It is described below an example of UI for setting the measurement conditions and the like of the X-ray diffraction system 100. It is assumed that the functions of the X-ray diffraction system 100 are implemented as software.

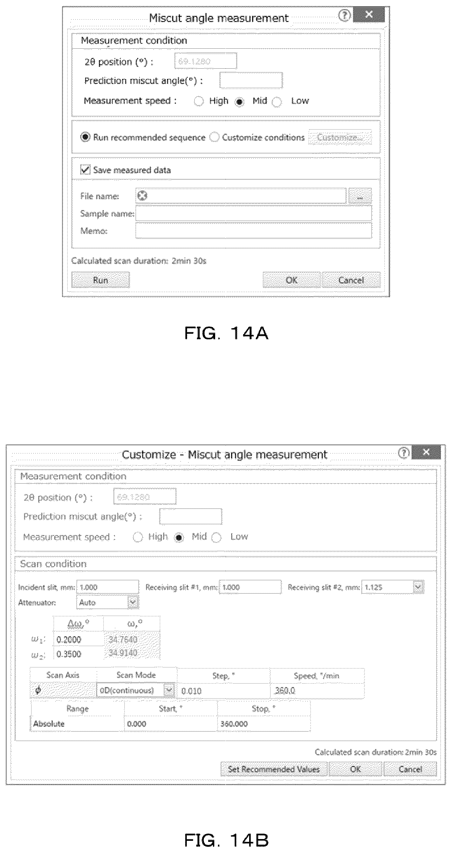

FIGS. 14A and 14B are schematic diagrams showing UI examples of entry dialogues when the software is executed. In UI shown in FIG. 14A, an angle of $2\theta$, a predicted miscut angle and a measurement speed can be set as the measurement conditions. It is also possible to set whether the measurement is performed in a recommended sequence or under customized conditions. In addition, FIG. 14B is an example of UI of an entry dialogue that is opened when the measurement conditions shown in FIG. 14A is customized. In UI shown in FIG. 14B, the parameters required for measurement, such as the angle of $2\theta$, the predicted miscut angle, measurement speed, slit-width of the incident side or the receiving side, type of attenuator, $\omega_1$ or $\omega_2$, scan mode, scan step, scan speed, scan range, start position and end position of the scan, can be set as the measurement conditions, respectively.

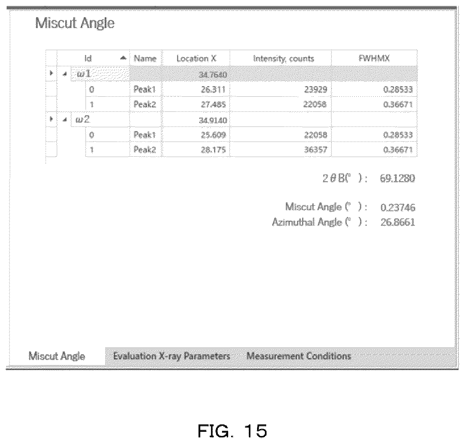

FIG. 15 is a schematic diagram showing an example of a measurement result output screen of the software. In the output window shown in FIG. 15, the angles of $\omega_1$ and $\omega_2$, the angles of the peak positions relative to them, the measured X-ray intensities, the full width at half maximum of the peaks, the angles of $2\theta$, the calculated miscut angles and azimuth angles are output.

Note that the setting items shown in FIG. 14A or 14B are exemplary, and even when the user sets them, only a part of them may be set, or all of them may be set. There may be sets or functions that are not shown in FIG. 14A or 14B. The measurement result and the like shown in FIG. 15 are examples, and only a part of them may be displayed. In FIG. 15, a measurement result or the like that is not displayed may be displayed.

[Calculation Method of Miscut angle and Azimuth Angle]

Next, a specific procedure for applying the method of the present invention to the X-ray diffraction system 100 is described.

(Description of Flow When Calculating only Miscut angle)

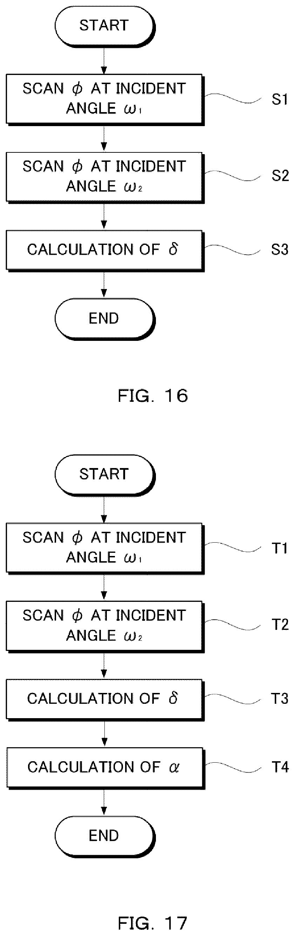

FIG. 16 is a flowchart showing an example of the operation of the X-ray diffraction system 100. FIG. 16 shows an example of the operation in the case where only the miscut angle is calculated. As a preliminary step, a single-crystal solid sample is placed on the sample stage 240 of the X-ray diffraction apparatus 200, after the sample surface is moved so as to come to the measurement reference position, the in-plane rotation axis $\varphi$ of the sample stage 240 and the sample surface normal axis are aligned. As a method of aligning the in-plane rotation axis $\varphi$ of the sample stage 240 with the sample surface normal axis, a known method may be used. The methods of the present invention described above may be used.

Next, the X-ray diffraction system 100 performs $\varphi$ scan at the incident angle $\omega_1$ (step S1). That is, the X-ray diffraction system 100, based on the control of the control section 310 of the control apparatus 300, makes the X-ray incident on the sample while fixing the incident angle to $\omega_1$, drives the goniometer 230 to rotate the sample about the in-plane rotational axis $\varphi$ with one rotation, measures the diffracted X-rays generated from the sample and measures 1 or 2 peak positions of $\varphi$. Next, the X-ray diffraction system 100 performs $\varphi$ scan at the incident angle $\omega_2$ (step S2). That is, the X-ray diffraction system 100, based on the control of the control section 310 of the control apparatus 300, makes the X-ray incident on the sample while fixing the incident angle to $\omega_2$, drives the goniometer 230 to rotate the in-plane rotational axis $\varphi$ with one rotation, measures the diffracted X-rays generated from the sample and measures 1 or 2 peak positions of $\varphi$. If two peak positions of $\varphi$ are measured in the measurement at $\omega_1$, the measurement may be terminated at a stage where one peak position of $\varphi$ is measured in the measurement at $\omega_2$. Thus, the measurement time can be shortened.

The incidence angles $\omega_1$ and $\omega_2$ are set to different values in the range of $\theta-\delta$ or more and $\theta+\delta$ or less. Here, $\theta$ indicates the Bragg angle of the crystal lattice plane of interest, and $\delta$ indicates an angle formed by the in-plane rotation axis of the sample stage and the crystal lattice plane normal of the sample. The incident angles $\omega_1$ and $\omega_2$ may be set by user input. In addition, the control apparatus 300 may acquire information about the sample and set them automatically. When calculating $\delta$ or $\alpha$ for multiple samples of the same kind, the incident angles $\omega_1$ and $\omega_2$ may be set and fixed in advance.

Then, the calculation section 350 calculates $\delta$ from $\omega_1$, $\omega_2$ and the peak positions of $\varphi$ obtained by the measurement (step S3). The calculation section 350 outputs the result as necessary, and the process ends. A configuration may be adopted in which only the result is stored and output when an instruction is given from the user. Since the in-plane rotation axis $\varphi$ of the sample stage 240 coincides with the sample surface normal axis, the calculated $\delta$ is the miscut angle of the sample.

In the step of calculating $\delta$ in the step S3 of the flowchart shown in FIG. 16, $\delta$ may be obtained by substituting three of the combinations of $\omega_1$, $\omega_2$ and the peak positions of $\varphi$ into the above formula (11) and solving the simultaneous equations. Since the number of unknowns is three of $\delta$, $\alpha$, and $\theta$ when calculating $\delta$ or $\alpha$, the calculation amount can be reduced rather than using four. The same applies to FIGS. 17 to 19.

Further, in the step of calculating $\delta$ in the step S3 of the flowchart shown in FIG. 16, $\delta$ may be obtained by substituting four of the combinations of $\omega_1$, $\omega_2$ and the peak positions of $\varphi$ into the above formula (11) and solving the simultaneous equations. The use of four of the combinations occurs an overdetermined system, but by using four of the combinations, the effect of measurement errors and the like can be reduced, and the calculated $\delta$ or $\alpha$ can be brought close to a true value. The same applies to FIGS. 17 to 19.

(Description of Flow when Calculating Miscut angle and Azimuth angle)

FIG. 17 is a flowchart showing a modified example of the operation of the X-ray diffraction system 100. FIG. 17 shows an example of the operation in the case of calculating the miscut angle and the azimuth angle. In the following description of the flowchart, the characteristic operation is described in detail, and the description of the operation already described may be omitted. The preparation is performed in the same manner as described above, and $\varphi$ scan is performed at the incident angle $\omega_1$ (step T1). Next, $\varphi$ scan is performed at the incident angle $\omega_2$ (step T2). Next, $\delta$ is calculated (step T3). The process from the preparation stage to the step T3 is the same as the process from the preparation stage to the step S3 described above.

Then, the calculation section 350 calculates $\alpha$ (step T4). The calculation section 350 outputs the result as necessary, and the process ends. The step T3 and the step T4 may be performed in any order or simultaneously. Since the in-plane rotation axis $\varphi$ of the sample stage 240 and the sample surface normal axis are aligned, the calculated $\delta$ and $\alpha$ are the miscut angle and the azimuth angle of the sample, respectively.

(Description Flow when Calculating Miscut angle when Miscut angle is Expected to be Small)

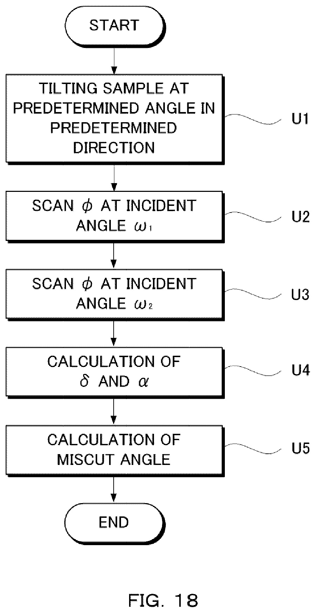

FIG. 18 is a flowchart showing a modified example of the operation of the X-ray diffraction system 100. FIG. 18 shows an example of an operation in a case where the miscut angle is calculated in a case where the miscut angle is expected to be small. After the same preparation step, the X-ray diffraction system 100 tilts the sample at a predetermined angle based on the control of the control section 310 of the control apparatus 300 (step U1). At this time, the control apparatus 300 stores the predetermined angle and the direction thereof. The process from the subsequent step U2 to the step U4 is the same as the above-described steps T1 to step T4. The values $\delta$ and $\alpha$ obtained in step U4 are calculated based on the values measured with tilting the sample, and therefore differ from the actual miscut angle and azimuth angle.

Next, the calculation section 350 calculates a miscut angle based on $\delta$, $\alpha$, a predetermined direction and a predetermined angle (step U5). The calculation section 350 outputs the result as necessary, and the process ends. Thus, even when the miscut angle of the sample is small, the miscut angle can be calculated. In step U5, the azimuth angle of the sample may be calculated.

(Description of Flow When Determining Whether or not to Apply Method of Invention)

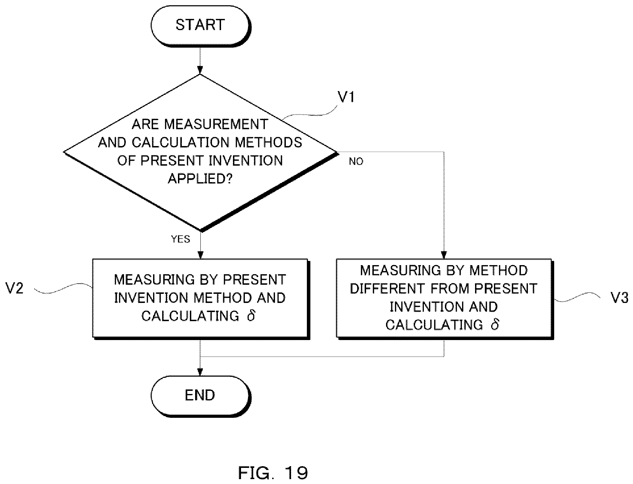

FIG. 19 is a flowchart showing a modified example of the operation of the X-ray diffraction system 100. FIG. 19 shows an example of an operation when determining whether to apply the method of the present invention. After the same preparation step as described above, the control apparatus 300 determines whether or not to apply the measurement and calculation method of the present invention according to the user's instruction or the property of the sample and determines the measurement and calculation method (step V1).

When it is determined in the step V1 that the measurement and calculation method of the present invention is to be applied (step V1-YES), the X-ray diffraction system 100 performs the measurement with the method of the present invention and calculates $\delta$ (step V2). In step V2, for example, any of the above flowcharts can be applied. On the other hand, when it is determined in step V1 that the measurement and calculation method of the present invention are not to be applied (step V1-NO), the X-ray diffraction system 100 performs the measurement with a method other than the present invention and calculates $\delta$ (step V3). As in step V3, for example, conventional measurement and calculation methods such as the $\varphi$ 4-point method can be applied. In both of steps V2 and V3, the calculation section 350 outputs the result as necessary, and the process ends. Accordingly, it is possible to change the measurement and calculation method according to the nature of the sample, and it is possible to appropriately reduce the total time for calculating the miscut angle for each of the plurality of samples.

[Example]

Using the X-ray diffraction system 100 configured as described above, a pseudo offset was given in advance for Si wafer using a Rx axis mechanism that controls the tilt of the sample stage. In this state, by the method of the present invention and the $\varphi$ 4-point method which is a conventional method, the X-ray diffraction intensity was measured, and the miscut angle and the azimuth angle were calculated.

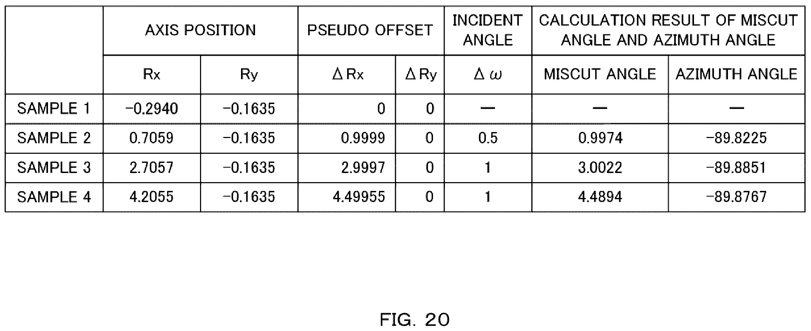

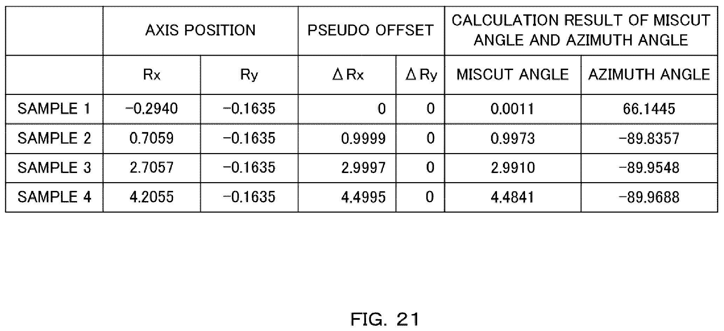

FIG. 20 is a table showing the values of the pseudo offset and the calculation results of the miscut angle and the azimuth angle calculated by the method of the present invention. FIG. 21 is a table showing the values of the pseudo offset

**EP 4 607 183 A1**

and the calculation results of the miscut angle and the azimuth angle calculated by the $\varphi$ 4-point method. For sample 3 with a pseudo offset of about 3° and sample 4 with a pseudo offset of about 4.5°, the miscut angle and the azimuth angle were able to be calculated with the same accuracy as the $\varphi$ 4-point method when the incident angle $\omega$ was set to $\theta \pm 1°$ with respect to the theoretical $\theta$.

For sample 1 in which the pseudo offset was about 0°, since the peak of $\varphi$ did not appear when the incident angle $\omega$ was set to $\theta \pm 1°$, the miscut angle and the azimuth angle could not be calculated. However, from the results of the samples 3 and 4, it is possible to perform the measurement and calculation for the sample as same as sample 3 and 4 by tilting the sample in advance even in such a case. Further, the accuracy of $\delta$ or $\alpha$ of the samples 3 and 4 is sufficiently high. Therefore, it has been found that the miscut angle and the azimuth angle can be calculated with the same accuracy as with that in the $\varphi$ 4-point method even when the miscut angle is small.

For sample 2 in which the pseudo offset is about 1 °, the miscut angle and the azimuth angle could not be calculated when the incident angle $\omega$ is set to $\theta \pm 1$ °, but the miscut angle and the azimuth angle could be calculated with the same accuracy as that in the $\varphi$ 4 point method when the incident angle $\omega$ is set to $\theta \pm 0.5$ °.

Further, the time required for the measurement for calculating $\delta$ by the method of the present invention was about half the time required for the measurement for calculating $\delta$ by the $\varphi$ 4-point method. In both methods, the calculation time of $\delta$ was negligible in comparison with the measurement time.

From the above results, according to the method for calculating the miscut angle of the single-crystal solid sample, the X-ray diffraction system and the program in the present invention, it was confirmed that the miscut angle and the azimuth angle can be calculated by using the data of the peak position of the diffracted X-ray intensity when performing two $\varphi$ scans with different incident angles of $\omega$ with the X-ray diffraction apparatus. In addition, it was confirmed that the measurement time required for this could be shorter than that of the conventional method.

The functionality of the elements disclosed herein may be implemented using circuitry or processing circuitry which includes general purpose processors, special purpose processors, integrated circuits, ASICs("Application Specific Integrated Circuits"), FPGAs("Field Programmable Gate Arrays"), conventional circuitry and/or combinations thereof which are programmed, using one or more programs stored in one or more memories, or otherwise configured to perform the disclosed functionality. Processors are considered processing circuitry or circuitry as they include transistors and other circuitry therein. The processor may be a programmed processor which executes a program stored in a memory. In the disclosure, the circuitry, units, or means are hardware that carry out or are programmed to perform the recited functionality. The hardware may be any hardware disclosed herein which is programmed or configured to carry out the recited functionality.

This application claims priority from Japanese Patent Application No. 2024-027017, filed on Feb. 26, 2024, the entire contents of Japanese Patent Application No. 2024-027017 are incorporated herein by reference.

Description of Symbols

100 X-ray diffraction system
200 X-ray diffraction apparatus
210 X-ray generating section
220 incident side optical unit
230 goniometer
240 sample stage
250 emitting side optical unit
260 detector
300 control apparatus
310 control section
320 apparatus information storing section
330 measurement data storing section
340 display section
350 calculation section
510 input device
520 display device

**Claims**

1. A method for calculating an angle $\delta$ formed by an in-plane rotational axis $\varphi$ of a sample stage and a crystal lattice plane normal axis of a single-crystal solid sample disposed on the sample stage, comprising the steps of:

when a Bragg angle corresponding to the crystal lattice plane is referred to as $\theta$,
making an X-ray incident on the sample at a first incident angle $\omega_1$ of $\theta$-$\delta$ or more and $\theta$+$\delta$ or less, measuring 1 or 2 peak positions of $\varphi$ by measuring a diffracted X-ray intensity while rotating the sample in-plane with the $\varphi$ as a central axis,
making an X-ray incident on the sample at a second incident angle $\omega_2$ of $\theta$-$\delta$ or more and $\theta$+$\delta$ or less different from the first incident angle $\omega_1$, measuring 1 or 2 peak positions of $\varphi$ by measuring a diffracted X-ray intensity while rotating the sample in-plane with the $\varphi$ as a central axis, and
calculating the $\delta$ from the $\omega_1$, the $\omega_2$ and the peak positions of the $\varphi$ obtained by the measurement.

2. The method according to claim 1,
wherein the step of calculating the $\delta$ comprises substituting three of the combinations of $\omega_1$, $\omega_2$ and the peak positions of $\varphi$ into the following formula (1), where $\alpha$ is an azimuth angle, and solving simultaneous equations to calculate the $\delta$:

$$\cos \omega \cos(\varphi - \alpha + 180) \sin \delta + \sin \omega \cos \delta = \sin \theta \qquad \cdots \ (1)$$

3. The method according to claim 1,
wherein the step of calculating the $\delta$ comprises substituting four of the combinations of $\omega_1$, $\omega_2$ and the peak positions of $\varphi$ into the following formula (2), where $\alpha$ is an azimuth angle, and solving simultaneous equations to calculate the $\delta$:

$$\cos \omega \cos(\varphi - \alpha + 180) \sin \delta + \sin \omega \cos \delta = \sin \theta \qquad \cdots \ (2)$$

4. The method according to claim 2 or 3, further comprising the step of calculating the $\alpha$.

5. The method according to any one of claims 1 to 4,

   wherein the $\varphi$ coincides with the sample surface normal axis of the sample, and
   the $\delta$ is a miscut angle formed by a crystal lattice plane normal axis of the sample and a sample surface normal axis of the sample.

6. The method according to claim 4,

   wherein the $\varphi$ coincides with the sample surface normal axis of the sample, and the method further comprises the step of tilting the sample at a predetermined angle in a predetermined direction from the state that the $\varphi$ coincides with the sample surface normal axis of the sample,
   wherein the step of calculating the $\delta$ comprises using the calculated $\delta$ and $\alpha$ as a temporary value and calculating a miscut angle formed by the crystal lattice plane normal axis of the sample and the sample surface normal axis of the sample based on the $\delta$, $\alpha$, the predetermined direction and the predetermined angle.

7. The method according to any one of claims 1 to 6, further comprising the step of determining a measurement and calculation method according to a user's instruction or a property of the sample.

8. A method for calculating an angle $\delta$ formed by an in-plane rotation axis $\varphi$ of a sample stage and a sample surface normal axis of a sample disposed on the sample stage, comprising the steps of:

   when a total reflection critical angle of the sample is referred to as $\theta$c, and a certain angle of more than 0° and the $\theta$c or less is referred to as $\theta$,
   making X-rays incident on the sample at a first incident angle $\omega_1$ of $\theta$-$\delta$ or more and $\theta$+$\delta$ or less, measuring 1 or 2 peak positions of $\varphi$ by measuring a reflected X-ray intensity while rotating the sample in-plane with the $\varphi$ as a central axis,
   making X-rays incident on the sample at a second incident angle $\omega_2$ of $\theta$-$\delta$ or more and $\theta$+$\delta$ or less different from the first incident angle $\omega_1$, measuring 1 or 2 peak positions of the $\varphi$ by measuring a reflected X-ray intensity while rotating the sample in-plane with the $\varphi$ as the center axis, and
   calculating the $\delta$ from the $\omega_1$, the $\omega_2$ and the peak positions of the $\varphi$ obtained by the measurement.

9. An X-ray diffraction system comprising;

an X-ray diffraction apparatus comprising an X-ray generating section for generating X-rays, a detector for detecting X-rays, a sample stage having an in-plane rotation axis $\varphi$ and a goniometer for controlling rotation of a sample, and

a control apparatus comprising a control section for controlling the X-ray diffraction apparatus, a calculation section for performing calculation based on measurement data measured with the X-ray diffraction apparatus, wherein the X-ray diffraction system calculates an angle $\delta$ formed by the in-plane rotation axis $\varphi$ of the sample stage and a crystal lattice plane normal axis of a single-crystal solid sample disposed on the sample stage, when a Bragg angle corresponding to the crystal lattice plane is referred to as $\theta$, the control section makes an X-ray incident on the sample at a first incident angle $\omega_1$ of $\theta-\delta$ or more and $\theta+\delta$ or less and controls the X-ray diffraction apparatus to measure 1 or 2 peak positions of $\varphi$ by measuring a diffracted X-ray intensity while rotating the sample in-plane with the $\varphi$ as a central axis,

the control section makes an X-ray incident on the sample at a second incident angle $\omega_2$ of $\theta-\delta$ or more and $\theta+\delta$ or less different from the first incident angle $\omega_1$ and controls the X-ray diffraction apparatus to measure 1 or 2 peak positions of $\varphi$ by measuring a diffracted X-ray intensity while rotating the sample in-plane with the $\varphi$ as a central axis, and

the calculation section calculates the $\delta$ from the $\omega_1$, the $\omega_2$ and the peak positions of the $\varphi$ obtained by the measurement.

10. A program for calculating an angle $\delta$ formed by an in-plane rotation axis $\varphi$ of a sample stage and a crystal lattice plane normal axis of a single-crystal solid sample disposed on the sample stage by controlling an X-ray diffraction apparatus comprising an X-ray generation section for generating X-rays, a sample stage having an in-plane rotation axis $\varphi$, and a goniometer for controlling the rotation of the sample, causing a computer to execute the processes of:

when a Bragg angle corresponding to the crystal lattice plane is referred to as $\theta$,

making an X-ray incident on the sample at a first incident angle $\omega_1$ of $\theta-\delta$ or more and $\theta+\delta$ or less, making the X-ray diffraction apparatus measure 1 or 2 peak positions of $\varphi$ by measuring a diffracted X-rays intensity while rotating the sample in-plane with the X-ray as a central axis,

making an X-ray incident on the sample at a second incident angle $\omega_2$ of $\theta-\delta$ or more and $\theta+\delta$ or less different from the first incident angle $\omega_1$, and making the X-ray diffraction apparatus measure 1 or 2 peak positions of $\varphi$ by measuring a diffracted X-ray intensity while rotating the sample in-plane with the $\varphi$ as a central axis, and

calculating the $\delta$ from the $\omega_1$, the $\omega_2$ and the peak positions of the $\varphi$ obtained by the measurement.

MISCUT ANGLE

SAMPLE SURFACE

CRYSTAL LATTICE PLANE (hkl)

FIG. 1

SAMPLE SURFACE
NORMAL

CRYSTAL LATTICE
PLANE NORMAL

MISCUT ANGLE

AZIMUTH ANGLE

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14A

FIG. 14B

## Miscut Angle

| | Id | ▲ | Name | Location X | Intensity, counts | FWHMX |
|---|---|---|---|---|---|---|
| ▸ ◢ | $\omega 1$ | | | 34.7640 | | |
| | 0 | | Peak1 | 26.311 | 23929 | 0.28533 |
| | 1 | | Peak2 | 27.485 | 22058 | 0.36671 |
| ▸ ◢ | $\omega 2$ | | | 34.9140 | | |
| | 0 | | Peak1 | 25.609 | 22058 | 0.28533 |
| | 1 | | Peak2 | 28.175 | 36357 | 0.36671 |

$2\theta B(°)$ :  69.1280

Miscut Angle (° ) :  0.23746
Azimuthal Angle (° ) :  26.8661

Miscut Angle    Evaluation X-ray Parameters    Measurement Conditions

FIG. 15

START

SCAN $\phi$ AT INCIDENT
ANGLE $\omega_1$ — S1

SCAN $\phi$ AT INCIDENT
ANGLE $\omega_2$ — S2

CALCULATION OF $\delta$ — S3

END

FIG. 16

START

SCAN $\phi$ AT INCIDENT
ANGLE $\omega_1$ — T1

SCAN $\phi$ AT INCIDENT
ANGLE $\omega_2$ — T2

CALCULATION OF $\delta$ — T3

CALCULATION OF $\alpha$ — T4

END

FIG. 17

START

TILTING SAMPLE AT
PREDETERMINED ANGLE IN
PREDETERMINED
DIRECTION — U1

SCAN $\phi$ AT INCIDENT
ANGLE $\omega_1$ — U2

SCAN $\phi$ AT INCIDENT
ANGLE $\omega_2$ — U3

CALCULATION OF
$\delta$ AND $\alpha$ — U4

CALCULATION OF
MISCUT ANGLE — U5

END

FIG. 18

FIG. 19

| | AXIS POSITION | | PSEUDO OFFSET | | INCIDENT ANGLE | CALCULATION RESULT OF MISCUT ANGLE AND AZIMUTH ANGLE | |
|---|---|---|---|---|---|---|---|
| | Rx | Ry | $\Delta$Rx | $\Delta$Ry | $\Delta\omega$ | MISCUT ANGLE | AZIMUTH ANGLE |
| SAMPLE 1 | −0.2940 | −0.1635 | 0 | 0 | — | — | — |
| SAMPLE 2 | 0.7059 | −0.1635 | 0.9999 | 0 | 0.5 | 0.9974 | −89.8225 |
| SAMPLE 3 | 2.7057 | −0.1635 | 2.9997 | 0 | 1 | 3.0022 | −89.8851 |
| SAMPLE 4 | 4.2055 | −0.1635 | 4.49955 | 0 | 1 | 4.4894 | −89.8767 |

FIG. 20

| | AXIS POSITION | | PSEUDO OFFSET | | CALCULATION RESULT OF MISCUT ANGLE AND AZIMUTH ANGLE | |
|---|---|---|---|---|---|---|
| | Rx | Ry | $\triangle$Rx | $\triangle$Ry | MISCUT ANGLE | AZIMUTH ANGLE |
| SAMPLE 1 | −0.2940 | −0.1635 | 0 | 0 | 0.0011 | 66.1445 |
| SAMPLE 2 | 0.7059 | −0.1635 | 0.9999 | 0 | 0.9973 | −89.8357 |
| SAMPLE 3 | 2.7057 | −0.1635 | 2.9997 | 0 | 2.9910 | −89.9548 |
| SAMPLE 4 | 4.2055 | −0.1635 | 4.4995 | 0 | 4.4841 | −89.9688 |

FIG. 21

European Patent Office
Europäisches Patentamt
European Patent Office
Office européen des brevets

# EUROPEAN SEARCH REPORT

**Application Number**

EP 25 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG JINXING ET AL: "Quick determination of included angles distribution for miscut substrate", MEASUREMENT, INSTITUTE OF MEASUREMENT AND CONTROL. LONDON, GB, vol. 89, 1 April 2016 (2016-04-01), pages 300-304, XP029551215, ISSN: 0263-2241, DOI: 10.1016/J.MEASUREMENT.2016.03.079 * page 301, left-hand column, paragraph 2 - page 303, right-hand column, paragraph 5; figures; tables * | 1-10 | INV. G01N23/207 |
| X | MCELHINNY KYLE M ET AL: "Graphene-induced Ge (001) surface faceting", SURFACE SCIENCE, NORTH-HOLLAND, AMSTERDAM, NL, vol. 647, 8 January 2016 (2016-01-08), pages 90-95, XP029605327, ISSN: 0039-6028, DOI: 10.1016/J.SUSC.2015.12.035 | 1,8-10 | |
| A | * page 91, left-hand column, paragraph 2 - page 95, left-hand column; figures; tables * | 2-7 | **TECHNICAL FIELDS SEARCHED (IPC)** G01N |
| A | CHEN Y ET AL: "THE IMPACT OF CRYSTAL CUT ERROR ON THE MEASURED IMPURITY PROFILES RESULTING FROM ION IMPLANTATION", IEEE TRANSACTIONS ON SEMICONDUCTOR MANUFACTURING, IEEE SERVICE CENTER, PISCATAWAY, NJ, US, vol. 13, no. 2, 1 May 2000 (2000-05-01), pages 243-248, XP000945973, ISSN: 0894-6507, DOI: 10.1109/66.843640 * the whole document * | 1-10 | |

The present search report has been drawn up for all claims

| Place of search | Date of completion of the search | Examiner |
|---|---|---|
| The Hague | 6 June 2025 | Savage, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 607 183 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S57136151 A
- JP H03019250 A
- JP 2006053141 A
- JP 2024027017 A